(19) Europäisches Patentamt
European Patent Office
Office européen des brevets

(11) EP 2 522 322 B1

## (12) EUROPEAN PATENT SPECIFICATION

(45) Date of publication and mention
of the grant of the patent:
**12.09.2018 Bulletin 2018/37**

(21) Application number: **10842160.3**

(22) Date of filing: **03.12.2010**

(51) Int Cl.:
***A61F 13/15*** *(2006.01)*   ***A61F 13/49*** *(2006.01)*
***B05C 5/02*** *(2006.01)*   ***B05B 12/08*** *(2006.01)*

(86) International application number:
**PCT/JP2010/071638**

(87) International publication number:
**WO 2011/083645 (14.07.2011 Gazette 2011/28)**

(54) **FLUID JET APPARATUS**

FLÜSSIGKEITSDÜSENVORRICHTUNG

APPAREIL À JET DE FLUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.01.2010 JP 2010003123**

(43) Date of publication of application:
**14.11.2012 Bulletin 2012/46**

(73) Proprietor: **Unicharm Corporation
Ehime 799-0111 (JP)**

(72) Inventor: **NAKANO, Takumi
Kanonji-shi
Kagawa 769-1602 (JP)**

(74) Representative: **Staeger & Sperling
Partnerschaftsgesellschaft mbB
Sonnenstraße 19
80331 München (DE)**

(56) References cited:
**EP-A2- 1 591 170     JP-A- 5 111 658
JP-A- 7 228 036      JP-A- 58 156 369
JP-A- 2003 211 059   US-A1- 2006 108 513**

## Description

Technical Field

The invention relates to a fluid discharging device which is used for manufacturing an absorbent article associated with a disposable diaper and the like, the fluid discharging device discharging fluid such as hot-melt adhesive towards a continuous sheet member made of nonwoven fabric and the like.

Background Art

Conventionally, in a manufacturing line of disposable diapers and the like, while continuously conveying in a transporting direction a continuous sheet member such as nonwoven fabric and the like, hot-melt adhesive is intermittently applied to the continuous sheet member by discharging the adhesive in the transporting direction ([PTL 1, 2, 3]).

Citation List

[Patent Literature].

[PTL 1] Japanese Patent Application Laid-open Publication No. 6-237 957
[PTL 2] EP 1 591 170 A2
[PTL 3] US 2006/0108513 A1

Summary of the Invention

Technical Problem

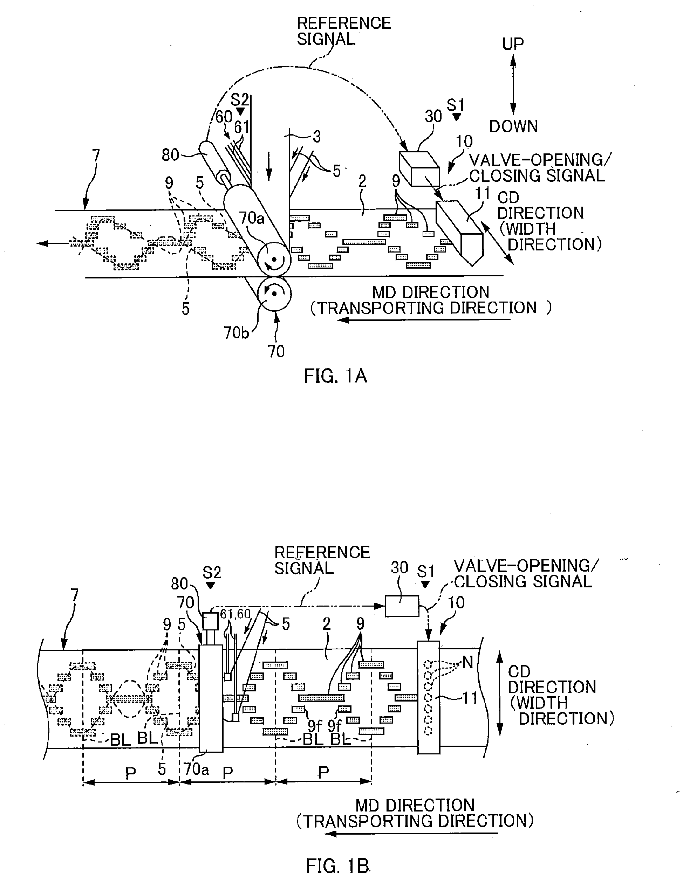

This intermittent application is performed by a hot-melt adhesive applying device 10 (hereinafter referred to as HMA applying device 10) (FIGS. 1A and 1B). The HMA applying device 10 includes a head 11 which is, for example, arranged at a certain position in the transporting direction, and the head 11 includes a plurality of nozzles N, N ... lined up in the width direction of a continuous sheet member 2. Corresponding to each of the nozzles N, one valve 14 (not shown in FIGS. 1A and 1B) is disposed. The valves 14 open and close under control of the controller 30 depending on an amount of transportation of the continuous sheet member 2, and thereby the nozzles N intermittently discharges hot-melt adhesive towards the continuous sheet member 2.

In order to control opening and closing operations of the valves 14 depending on transportation amount, an encoder 80 is used, for example. The encoder 80 is configured, for example, so as to repeatedly output a digital value from 0 to 8191 during a period in which the continuous sheet member 2 is transported by an amount corresponding to a product pitch P (the length P of one product) of the diaper, the digital value being proportional to the amount by which the continuous sheet member 2 is transported; the product pitch P being defined on the continuous sheet member 2. The controller 30 controls opening and closing operations of the valves 14 as follows: open the valves 14 when a digital value transmitted from the encoder 80 reaches the predetermined first regulation value; close the valves 14 when the digital value reaches the predetermined second regulation value; and the like. The first regulation value, the second regulation value, etc are set up respectively for the valves 14.

By the way, because the tips of the nozzles N are opposite the continuous sheet member 2 with a certain spacing, it always takes a certain period of time for hot-melt adhesive that is discharged from the tips of the nozzles N to land on the continuous sheet member 2. Further, the transport velocity V2 of the continuous sheet member 2 varies considerably during manufacturing. Therefore, it is possible that the variation of the transport velocity V2 causes the actual applying position of the hot-melt adhesive on the continuous sheet member 2 to displace in the transporting direction from the target position.

As a method for suppressing the foregoing displacement of the applying position from the target position, the foregoing first and second regulation values may be compensated based on the compensation values which are obtained by the controller 30 by executing operations based on the transport velocity V2 of the continuous sheet member 2. In this case, the controller 30 repeats the operation for the foregoing compensation with a certain control period Tc.

However, if the operations are executed for each of the valves 14, 14, ... of the head 11 individually, the load of the operations increases considerably. As a result, it is possible that the load exceeds the processing capability of the controller 30.

Further, if the foregoing compensation is performed for each of the valves 14, 14, ... individually, it is necessary to prepare, for each of the valves 14, 14, ... , some compensation data such as a compensation-value table which represents the relationship between the transport velocity V2 and the compensation value H, individually. Therefore, it takes considerable time and effort to prepare the compensation data.

The invention has been made in view of the above conventional problems, and an advantage thereof is to provided a fluid discharging device which can reduce an operational load of a controller associated with compensation values and which can also reduce considerable time and effort to prepare the compensation data which is used for calculating the compensation values.

Solution to Problem

An aspect of the invention to achieve the above advantage is a fluid discharging device that discharges fluid from a plurality of discharge openings towards a continuous sheet member which is continuously transported in a transporting direction, the discharge openings being arranged in a width direction of the continuous sheet member associated with an absorbent article, including:

a plurality of valves that correspond to the discharge openings and intermittently discharge the fluid from the discharge openings by opening and closing operations; and
a controller that controls the opening and closing operations of each of the valves individually depending on a transportation amount of the continuous sheet member, and a memory that stores a shared compensation value that is represented by a value indicating the transportation amount, wherein
the controller calculates the shared compensation value based on a transport velocity of the continuous sheet member, and
the controller shifts an open-close timing of at least several valves of the plurality of valves from a previously-determined open-close timing, based on the shared compensation value.

Other features of this invention will become apparent from the description in this specification and the attached drawings.

Effects of the Invention

According to the invention, it is possible to reduce an operational load of a controller associated with compensation values and also to reduce considerable time and effort to prepare compensation data which is used for calculating the compensation values.

BRIEF DESCRIPTION OF THE DRAWINGS

FIG. 1A is a schematic perspective view of a manufacturing line including an HMA applying device 10 according to the first reference example, and FIG. 1B is a plan view of the same.
FIG. 2 is a schematic diagram of the configuration of HMA applying device 10.
FIG. 3 is a longitudinal sectional view of a head 11 of the HMA applying device 10.
FIG. 4 is a plan view of a continuous sheet member 2, which is for describing input values of a first regulation value and a second regulation value.
FIGS. 5A and 5B are diagrams showing how the application pattern of hot-melt adhesive is formed displaced in its entirety in the MD direction based on an adjustment value Ya.
FIG. 6 is a graph showing a relationship between the transport velocity V2 of the continuous sheet member 2 and a position at which hot-melt adhesive discharged from the head 11 lands on the sheet member in the MD direction.
FIG. 7 is a graph showing a relationship between the transport velocity V2 and a compensation value H which is subject to a compensation according to the second embodiment.
FIG. 8 is a plan view of an HMA applying device 10 according to the third embodiment.

Mode for Carrying Out the Invention

At least the following matters will be made clear by the description in the present specification and the accompanying drawings.
A fluid discharging device that discharges fluid from a plurality of discharge openings towards a continuous sheet member which is continuously transported in a transporting direction, the discharge openings being arranged in a width direction of the continuous sheet member associated with an absorbent article, including:

a plurality of valves that correspond to the discharge openings and intermittently discharge the fluid from the discharge openings by opening and closing operations; and

a controller that controls the opening and closing operations of each of the valves individually depending on a transportation amount of the continuous sheet member, wherein

the controller calculates a shared compensation value that is represented by a value indicating the transportation amount, based on a transport velocity of the continuous sheet member, and

the controller shifts an open-close timing of at least several valves of the plurality of valves from a previously-determined open-close timing, based on the shared compensation value.

With such a fluid discharging device, the shared compensation value is used for the several valves. Therefore, in order to compensate at least the several valves, it is sufficient for the controller to obtain one shared compensation value which can be applied for all of these valves. This makes it possible to reduce the load of the controller of the operations associated with the compensation value.

Further, the shared compensation value is used for the several valves. Therefore, the compensation data used when obtaining the compensation value does not have to be prepared for each valve. This makes it possible to reduce considerable time and effort to prepare the compensation data.

In such a fluid discharging device, it is desirable that

for each of the opening operation and the closing operation, the controller has compensation data that denotes a relationship between the transport velocity and the shared compensation value,

in the opening operation, the controller obtains the shared compensation value for the opening operation of the several valves based on the compensation data for the opening operation, and

in the closing operation, the controller obtains the shared compensation value for the closing operation of the several valves based on the compensation data for the closing operation.

With such a fluid discharging device, even if the period of the valve opening operation is different from that of the closing operation, the compensation can be made properly for each operation.

In such a fluid discharging device, it is desirable that

the previously-determined open-close timing includes a previously-determined opening timing and a previously-determined closing timing,

the controller includes, for each of the valves,

a first regulation value that sets up the previously-determined opening timing and

a second regulation value that sets up the previously-determined closing timing,

the first regulation value and the second regulation value are represented by the value indicating the transportation amount, and

using the value indicating the transportation amount, the controller controls the opening and closing operations of each of the valves individually depending on the transportation amount.

With such a fluid discharging device, the previously-determined open-close timing can be set up for each of the valves. Therefore, by combining landing marks of fluid discharged towards the continuous sheet member from the discharge openings which respectively correspond to the several valves, any landing pattern of the fluid can be formed on the continuous sheet member.

Further, shifting the open-close timing of the several valves from the previously-determined open-close timing based on the shared adjustment value makes it possible to form the landing pattern which is displaced in the transporting direction of the continuous sheet member while substantially maintaining the shape of the landing pattern without deformation.

In such a fluid discharging device, it is desirable that

all of the plurality of valves are disposed in one head that includes the plurality of discharge openings, and

the number of the several valves is the same as the number of the plurality of valves.

With such a fluid discharging device, the shared compensation value is used for all of the valves which are disposed in one head. Therefore, the landing pattern which is formed with the fluid discharged from all of the discharge openings on the head can be displaced in the transporting direction while substantially maintaining the shape of the pattern.

In such a fluid discharging device, it is desirable that

one valve is provided for every discharge opening.

With such a fluid discharging device, the valves are provided respectively for the discharge openings. Therefore, a landing pattern of the fluid can be formed precisely on the continuous sheet member.

=== First Reference Example ===

FIG. 1A is a schematic perspective view of a manufacturing line including an HMA discharging device 10 according to the first reference example, and FIG. 1B is a plan view of the same.

On this manufacturing line, a continuous body 7, which is a half-completed product, is produced. For example, the continuous body 7 includes: a continuous sheet member 2 which serves as a top sheet of a diaper and is made of nonwoven fabric, etc; a continuous sheet member 3 which serves as a back sheet of the diaper and is made of nonwoven fabric, etc; and a pair of rubber threads 5 and 5 which are placed between these sheet members to form leg gathers.

That is, this manufacturing line includes a transporting apparatus (not shown) such as a roll, etc. which continuously conveys the continuous sheet member 2 in a transporting direction, which is the MD direction. On the conveying path, there are the two following sections: an HMA application section S1 in which hot-melt adhesive (hereinafter also referred to as HMA) is applied to the continuous sheet member 2 in such an application pattern as a pair of approximate sine curves; and a processing section S2 in which while the rubber threads 5 and 5 being continuously supplied towards an HMA applying area 9 on the continuous sheet member 2, the continuous sheet member 3 which becomes a back sheet is placed on and bonded to the continuous sheet member 2.

Hereinafter, a direction perpendicular to the MD direction is referred to as a CD direction; the CD direction is the same as the width direction of the continuous sheet members 2 and 3.

In the HMA application section S1, an HMA applying device 10 is installed. The HMA applying device 10 corresponds to "fluid discharging device" according to the invention, which will be described later.

In the processing section S2, there are installed a rubber-thread supply device 60 and pressing rolls 70 which are examples of a processing apparatus. The rubber-thread supply device 60 includes a pair of arms 61 and 61 which moves back and forth in the CD direction while supplying the rubber threads 5 and 5 in the MD direction. While moving back and forth once during a period in which the continuous sheet member 2 is transported by an amount corresponding to a product pitch P in the MD direction, the arms 61 and 61 supply the rubber threads 5 and 5 towards the nip of the pressing rolls 70. Thereby, the arms 61 and 61 place the rubber threads 5 and 5 on the continuous sheet member 2 in an arrangement pattern such as an approximate sine curve which is similar to the foregoing application pattern.

The pressing rolls 70 have a pair of upper and lower rolls 70a and 70b which are driven and rotate about a rotational axis pointing in the CD direction. To the nip of them, there are fed not only the foregoing continuous sheet member 2 but also the continuous sheet member 3 which becomes a back sheet. Therefore, the continuous sheet member 2 which becomes a top sheet and the continuous sheet member 3 which becomes a back sheet overlay each other having the rubber threads 5 and 5 between these two sheets 2 and 3; the threads and the sheets are pinched by the pair of rolls 70a and 70b and are crimped.

On the continuous sheet member 2 which becomes a top sheet, a layout of a plurality of diapers lined up in the MD direction at the product pitch P is planned. In other words, target positions where such various parts as the rubber threads 5 and 5 will be joined or processed are planned. In the first reference example, identification of which of the target positions in a diaper corresponds to the portion that is currently being processed by a processing apparatus is performed with reference to the pressing rolls 70. That is, it is possible to detect in real time which portion of the diaper is currently passing the pressing rolls 70 and which portion is being crimped thereby.

The detection is performed, for example, by a rotary encoder 80 which is disposed of the shaft end of one of the pressing rolls 70 in an integrated manner. Specifically, the encoder 80 outputs one of, for example, 8192 digital values (corresponding to the "value indicating the transportation amount") from 0 to 8191, during a period in which the continuous sheet member 2 is transported by an amount corresponding to the product pitch P, the digital value being proportional to the amount by which the sheet member 2 is transported; and the encoder 80 repeats it. In addition thereto, "0" of the digital values is defined to be associated with the boundary position BL of products adjacent in the MD direction. That is, when the boundary position BL passes through the nip of the pressing rolls 70, the encoder 80 outputs a digital value "0", and then outputs digital values successively from "1" to "8191" till the next boundary position BL passes.

These digital values are used, for example, as a reference signal for controlling the back-and-forth motion of the arms 61 and 61 of the rubber-thread supply device 60. That is, the rubber-thread supply device 60 includes: a servomotor (not shown) which moves the arms 61 and 61 back and forth in the CD direction; and a controller (not shown). The controller drives and controls the servomotor based on the digital values that are input from the encoder 80, and thereby the arms 61 and 61 move back and forth in the CD direction. More specifically, the controller recognizes which portion of the diaper is currently passing the nip of the pressing rolls 70, based on the digital value of the encoder 80; simultaneously, the controller causes the rubber threads 5 and 5 to move to and be arranged at the position where in the CD direction the rubber threads 5 are to be joined. Thereby, the rubber threads 5 and 5 are arranged at a position where in the individual product the rubber threads are to be processed. Hereinafter, the digital value is also referred to as a reference signal.

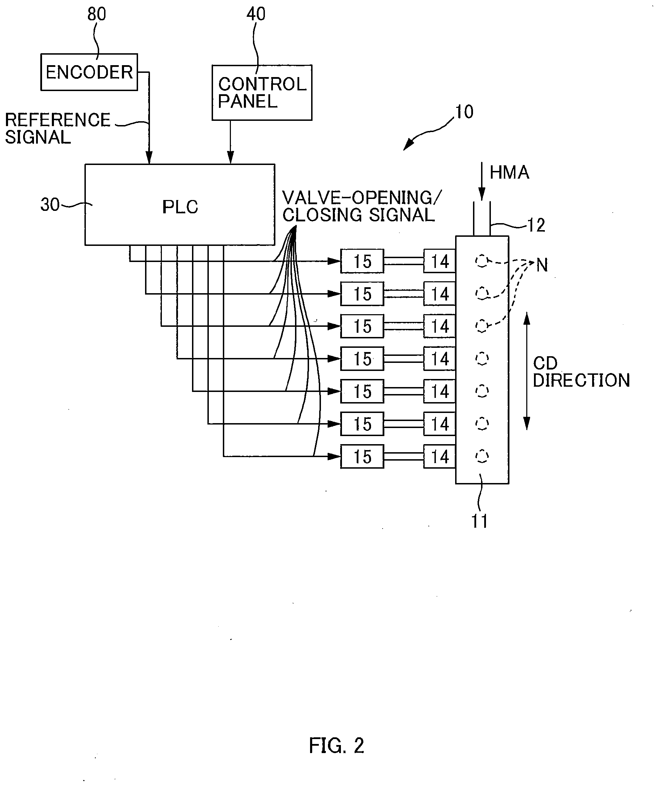

FIG. 2 is a schematic diagram of the configuration of the HMA applying device 10. This HMA applying device 10 includes a head 11, a PLC 30 (programmable logic controller) which serves as a controller, and a control panel 40.

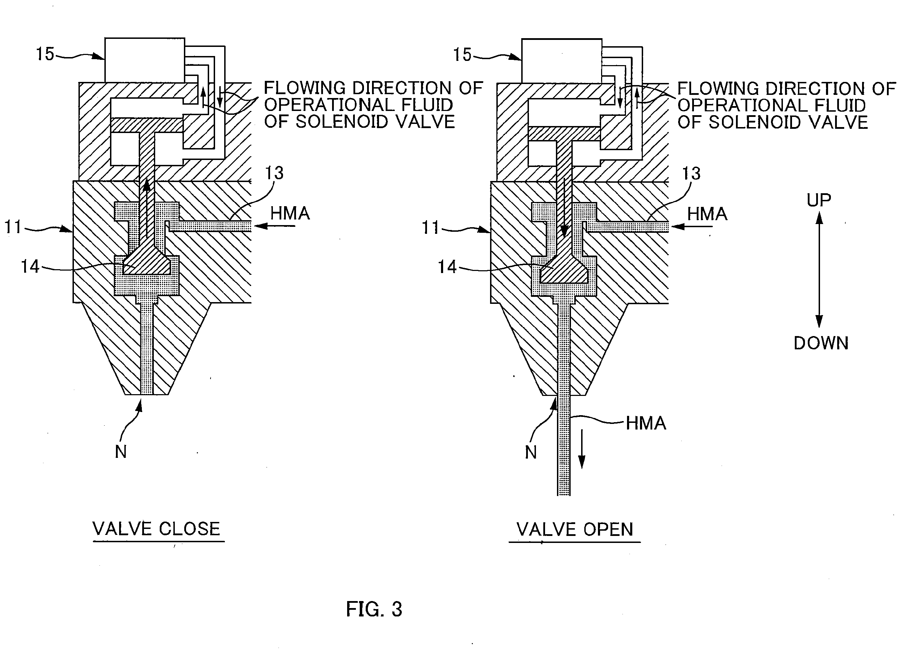

The head 11 has a plurality of (for example, 7) nozzles N (corresponding to the "discharge opening") lined up in the CD direction. Also, the head 11 is provided with one supply path 12 which supplies hot-melt adhesive to a flow path in the head 11. On the downstream side of the supply path 12, the flow path is divided into branches corresponding to the nozzles N; that is, one branch path 13 (FIG. 3) is formed for each nozzle N. As shown in the longitudinal sectional view of the head 11 in FIG. 3, each branch path 13 has a valve 14 which opens and closes the flow path corresponding to the branch path 13. Each valve 14 has a solenoid valve 15. To each solenoid valve 15, valve-opening/closing signal is sent from the PLC 30, and thereby, the solenoid valve 15 makes the corresponding valve 14 to open and close. The nozzles N intermittently discharge hot-melt adhesive towards the continuous sheet member 2. Therefore, as shown in FIG. 1B, band-like applying areas 9 along the MD direction are intermittently formed respectively for the nozzles N.

The PLC 30 sends a valve-opening/closing signal depending on the transportation amount of the continuous sheet member 2, at a timing which is determined for each of the valves. Therefore, the band-like applying areas 9, 9 ... formed by the nozzles N are all combined, resulting in the application pattern in which two approximate sine curves are lined up in the CD direction, as shown in FIG. 1B.

The valve-opening/closing signals are set, for example, by the control panel 40. The control panel 40 includes input buttons corresponding to the valves 14 for inputting the first regulation values, the second regulation values and the like, the first regulation values instructing the opening timing and the second regulation values instructing the closing timing of the valves 14. When a digital value that is input from the encoder 80 reaches the first regulation value, the PLC 30 sends the valve-opening signal to the solenoid valve 15. Also, when the digital value reaches the second regulation value, the PLC 30 sends the valve-closing signal to the solenoid valve 15. This makes the valves 14 to open and close.

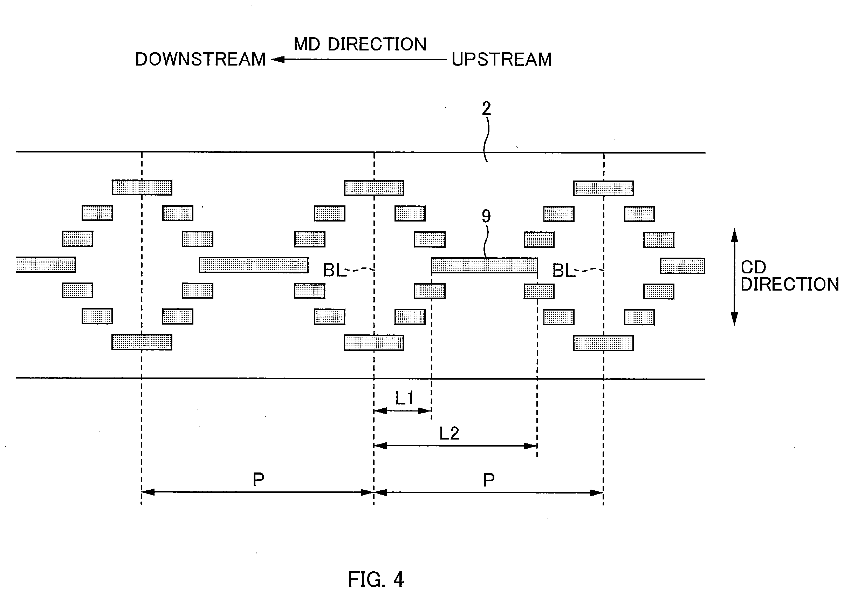

Input values of the foregoing first regulation values, second regulation values, etc are basically determined based on the product specification of the diaper. FIG. 4 is an explanatory diagram thereof, which is a plan view of the continuous sheet member 2. The description of the HMA applying area 9 at the center in the CD direction in FIG. 4 will be made below. The following distances are predetermined typically based on the product specification of the diaper: a distance from the downstream end of an HMA applying area 9 to the boundary position BL upstream in the MD direction of the diapers; and a distance from the upstream end of the applying area 9 to the boundary position BL. These distances are respectively referred to as L1 and L2. Basically, the input value of the first regulation value is obtained by the following formula (1), and the input value of second regulation value is obtained by the following formula (2).

$$\texttt{Input Value of First Regulation Value = L1 / P × 8192 ... (1)}$$

$$\texttt{Input Value of Second Regulation Value = L2 / P × 8192 ... (2)}$$

It should be noted that "P" of the foregoing formulas 1 and 2 is the product pitch P in the MD direction, that is, the total length of a diaper in the MD direction. Also, "8192" of the foregoing formulas 1 and 2 is the number of the digital values (0 to 8191) which the encoder 80 output during a period in which the sheet member 2 is transported by an amount corresponding to the product pitch P.

However, the hot-melt adhesive discharged based on the foregoing input values can land at a target applying area of a diaper precisely if the following relationship is satisfied: the length of the conveying path of the continuous sheet member 2 between the pressing rolls 70 and the nozzles N of the head 11 is an integral multiple of the product pitch P. This is because the digital value of the encoder 80, which are the reference signal, indicates which of the target positions in a diaper is currently processed at the position of the pressing rolls 70.

Further, even if the foregoing input values enable hot-melt adhesive to land at the target applying area precisely, it is possible that rearranging units in a periodic repair work of the manufacturing line or changing a product (including changing the size of the product (diaper)) causes change of the conveying path length of the continuous sheet member 2 between the pressing rolls 70 and the nozzles N of the head 11, and that the change causes a displacement of the actual applying area 9 from the target applying area in the MD direction under the condition of using the foregoing input values.

Therefore, each time when preparing the manufacturing line, an operator of the manufacturing line resets the foregoing regulation values. More specifically, the procedure is as follows. Firstly, while maintaining the foregoing regulation values at the same as the values from before the periodic repair work or the product change, hot-melt adhesive is discharged from the head 11 towards the continuous sheet member 2 which is being conveyed at a certain reference velocity Vb. Then, the operator measures the displacement δ of the actual applying area 9 of the adhesive on the continuous sheet member 2 from the target applying area. The measured displacement δ is converted into digital value of the encoder 80 by **the following formula 3,** and such values as the foregoing first and second regulation values are shifted by the conversion value Y of the displacement and these shifted values are input to the first and second regulation values. Thereby, the open-close timing of the valves 14 is adjusted.

$$\mathtt{Displacement\ Conversion\ Value\ Y = \delta / P \times 8192 \ ... \ (3)}$$

However, the foregoing adjustment for all the valves 14, 14, ... requires considerable time and effort. Further, if the cause is change of the conveying path length as mentioned above, the foregoing displacement conversion values Y of all the valves 14, 14, ... should be approximately the same.

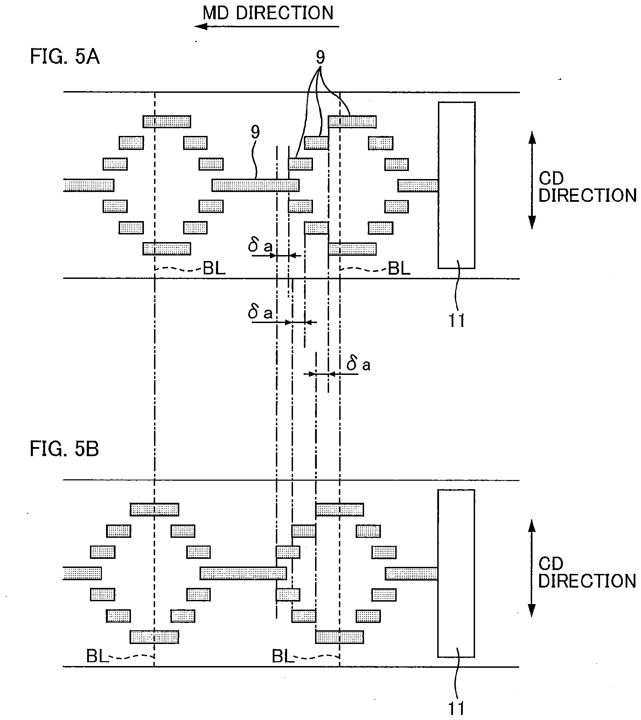

Therefore, in the first reference example, only one of the displacement conversion values Y mentioned above is input as an adjustment value Ya from the control panel 40. Then, the PLC 30 executes an operation so that the first and second regulation values are shifted by the adjustment value Ya, these values being associated with all the valves 14, 14, ... of the head 11. The shifted values, which are the operation results, are stored in a memory of the PLC 30 as a new first regulation value and a new second regulation value. Thereafter, the PLC 30 sends valve-opening/closing signals to the valves 14 based on such values as the new first regulation values and the new second regulation values which are reset respectively for the valves 14. Therefore, the application pattern of hot-melt adhesive is displaced by a length 5a (= Ya/8192 $\times$ P: corresponding to the adjustment value Ya) while substantially maintaining the shape of the pattern, from the state shown in FIG. 5A to the state shown in FIG. 5B.

In order to shift the open-close timing so that the application pattern is displaced downstream in the MD direction (that is, to advance the timing), an adjustment value Ya which is a negative value is added to the first and second regulation values. On the other hand, in order to shift the open-close timing so that the application pattern is displaced upstream (that is, to delay the timing), an adjustment value Ya which is a positive value is added. Whether the timing is advanced or delayed is determined by the displacement relationship in the MD direction between the target applying area and the actual applying area 9.

Further, in the foregoing example, the instruction values, such as the first regulation value, of the valve-opening/closing signal are shifted at the same time for all the valves 14, 14, ... by the adjustment value Ya; thereby the new first regulation value, etc are determined. However, unlike the foregoing example, the digital value which is output by the encoder 80 may be shifted by the adjustment value Ya. In this case, the PLC 30 compares the first regulation value, the second regulation value, and the like with the digital value which is shifted by the adjustment value Ya. Based on the comparison, the PLC 30 outputs the valve-opening signal and/or the valve-closing signal. In order to shift the open-close timing so that the application pattern is displaced downstream in the MD direction (that is, to advance the timing), an adjustment value Ya which is a positive value is added to the digital value. On the other hand, in order to shift the open-close timing so that the application pattern is displaced upstream (that is, to delay the timing), an adjustment value Ya which is a negative value is added.

=== Second Reference Example and First Embodiment ===

In addition to the first reference example mentioned above, the second reference example includes changing the discharging timing of the hot-melt adhesive (that is, the open-close timing of the valves 14) according to the transport velocity V2 of the continuous sheet member 2. The configuration except for this point is almost the same as the first reference example, the same description will be omitted.

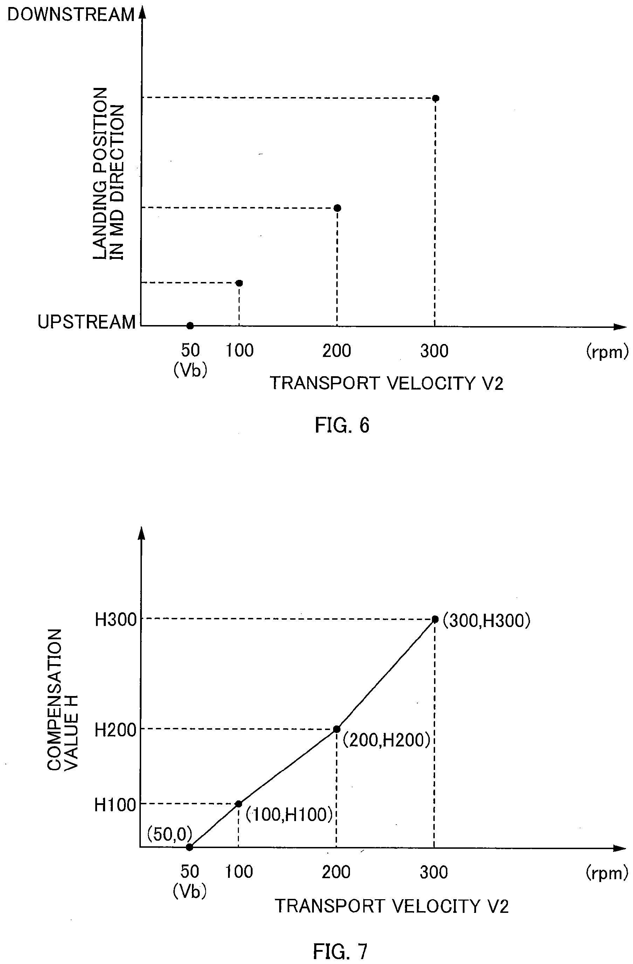

FIG. 6 is a graph showing a relationship between the transport velocity V2 of the continuous sheet member 2 and a position at which hot-melt adhesive discharged from the head 11 lands on the continuous sheet member 2. As can be seem from FIG. 6, the landing position is displaced more downstream in the MD direction, as the transport velocity V2 becomes greater. The reason is as follows: because the tips of the nozzles N are opposite the continuous sheet member 2 with a certain spacing, it always takes a certain period of time for hot-melt adhesive that is discharged from the tips of the nozzles N to land on the continuous sheet member 2; therefore, the continuous sheet member 2 moves greater during the certain time period as the transport velocity V2 becomes greater. Therefore, change of the transport velocity V2 of the continuous sheet member 2 causes variation of the HMA applying areas 9 in the MD direction.

Therefore, in the second reference example, in order to suppress the variation of the applying areas 9, the transport velocity V2 is measured in real time. And then, based on the measured value of the transport velocity V2, the PLC 30 performs successively a compensation of the values of the foregoing new first and/or second regulation values. The PLC 30 compares the compensated new first regulation value and new second regulation value with the digital value of the encoder 80. Based on the comparison, the PLC 30 sends the valve-opening/closing signal to the valves 14. The foregoing compensation and comparison are repeatedly performed with a control period Tc of a few milliseconds. This enables the PLC 30 to send the valve-opening/closing signal always at an appropriate timing, regardless of the transport velocity V2 which changes gradually. The measured value of the transport velocity V2 is sent instantly to the PLC 30 from a speedometer such as a pulse generator, etc (not shown), the speedometer being included in the vicinity of the pressing rolls 70 or the head 11.

FIG. 7 is a graph showing a relationship between the transport velocity V2 and a compensation value H which is subject to the foregoing compensation. In this example, the compensation value H is determined as follows: the slowest value of the transport velocity V2 in the manufacturing line is defined as the reference velocity Vb and the compensation value H associated with the reference velocity Vb is set to zero (a so-called reference value), for example. That is, assuming that a landing position at this reference velocity Vb is defined as a reference landing position, the compensation value H associated with various values of the transport velocity V2 are determined based on displacement $\delta1$ of the landing position from the reference landing position. Therefore, the foregoing compensation value H is calculated as follows. While actually transporting the continuous sheet member 2, for example, at a transport velocity V2 to which the corresponding compensation value H has to be obtained, hot-melt adhesive is discharged from the head 11. Then, the displacement 51 of its landing position from the reference landing position is measured, and the displacement $\delta1$ in the following formula 4 is substituted.

$$\text{Compensation Value H} = \delta1 \ / \ P \ \times \ 8192 \quad ... \quad (4)$$

Using the relationship shown in the graph of FIG. 7, the compensation of the foregoing new first and second regulation values is performed as follows.

Firstly, while successively receiving from the speedometer the transport velocity V2 which is measured in real time, the PLC 30 obtains the compensation value H corresponding to the received value of the transport velocity V2 based on the relationship of FIG. 7. The obtained compensation value H is subtracted from the foregoing new first and second regulation values. The values after the subtraction becomes the new first and second regulation values, and the first and second regulation values are updated.

It should be noted that, the relationship between the transport velocity V2 and the compensation value H shown in FIG. 7 is stored in the memory of the PLC 30 in a form of a compensation-value table consisting of a plurality of pairs of the transport velocity V2 and the corresponding compensation value H. For example, the compensation-value table can accommodate four data pairs of (V2, H): (50, 0), (100, H100), (200, H200), and (300, H300). A compensation value H corresponding to any value of the transport velocity V2 which is not stored in the compensation-value table is obtained by interpolation using two data pairs among the foregoing four pairs stored in the compensation-value table. For example, if the transport velocity V2 is between 200 (rpm) and 300 (rpm), the compensation value H corresponding to the velocity is obtained by linear interpolation based on the following formula 5.

$$H = (H300 - H200) \ / \ (300 - 200) \ \times \ (V2 - 200) \ + \ H200 \quad ...$$
$$(5)$$

The compensation-value table mentioned above is prepared for each of the valves 14. In addition, the table is prepared for each of the opening and closing operations of every valve 14. The reason the compensation-value table is prepared for each of the opening and closing operations is because the time it takes to open the valve 14 is sometimes different from the time it takes to close the valve 14.

In the first embodiment to be described below, the valves 14 of the nozzles N included in the head 11, a flow path from the valves 14 to the nozzles N, and the like are configured in the same manner over all the nozzles N, N .... In addition thereto, a distance between the continuous sheet member 2 and the tip of each nozzle N is set to the same over all the nozzles N, N .... Therefore, as for the operation of discharging hot-melt adhesive based on the valve-opening/closing signal, there is substantially no difference among the nozzles N, N, ... of the head 11.

In this first embodiment, all of the valves 14, 14, ... share the same compensation-value table, not each of the valves 14 has its own compensation-value table. That is, one compensation-value table for the opening operation and one compensation-value table for the closing operation are included, and this pair of the compensation-value tables are shared by all of the valves 14, 14, ....

In this case, it is sufficient that, in some cases such as preparing the manufacturing line, an operator obtains the foregoing data of the compensation-value table, that is, the plurality of data pairs (V2, H) mentioned above, for just any one of the valves 14 in the head 11. This can make the workload it takes for the operator to obtain the data of the compensation-value table considerably smaller than when obtaining the data for every valve.

Further, when the PLC 30 repeats its operations such as compensation with a certain control period Tc as mentioned above, as for the compensation operation it is not necessary to obtain a large number of compensation values H referring to the large number of compensation-value tables. As a result, an operational load of the PLC 30 can be reduced considerably. The more detailed description is as follows. With this method, when the PLC 30 sends the valve-opening signals to the valves 14, the compensation value H corresponding to the transport velocity V2 from the speedometers is obtained based on one compensation-value table for the opening operation. And then, the compensation value H is defined as a shared compensation value H (corresponding to the "shared compensation value") for all the valves 14, 14, ..., and the compensation value H is subtracted from the new first regulation value of each valve 14. The compensation for the opening operation is completed. In the same way, when the PLC 30 sends the valve-closing signals to the valves 14, the compensation value H corresponding to the transport velocity V2 from the speedometers is obtained based on one compensation-value table for the closing operation. And then, the compensation value H is defined as a shared compensation value H (corresponding to the "shared compensation value") for all the valves 14, 14, ..., and the compensation value H is subtracted from the new second regulation value of each valve 14. The compensation for the closing operation is completed. This makes it possible to reduce the operational load of the PLC 30.

=== Second Embodiment ===

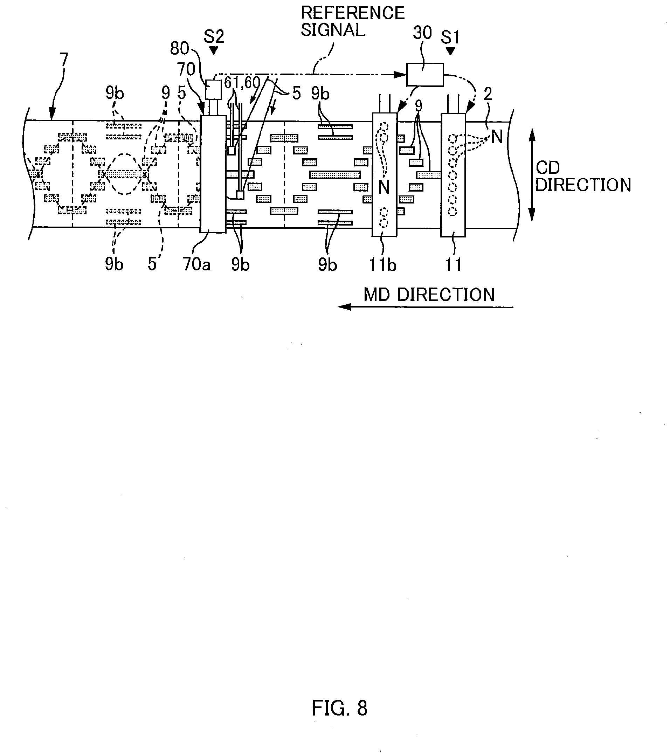

FIG. 8 is a plan view of an HMA applying device 10 according to the second embodiment. In the foregoing first reference example and first embodiment, the number of the head 11 of the HMA applying device 10 is one. However, in the second embodiment shown in FIG. 8, two heads 11 and 11b, as an example of a plurality of the heads 11, are arranged and their positions in the MD direction are different. In this example, the compensation-value tables, etc of the first embodiment are set up independently for each of the heads 11 and 11b.
That is, the PLC 30 can set up the first regulation value, the second regulation value, etc for each of the valves 14 included in the heads 11 and 11b; in addition, the compensation-value tables of the first embodiment and the adjustment value Ya can be set up for each of the heads 11 and 11b. Therefore, the adjustments of these heads 11 and 11b will not affect each other. That is, the open-close timing of the valves 14, 14, ... included in either one of the heads 11 and 11b can be shifted for all of the valves based on the adjustment value Ya and the compensation-value tables so that the application pattern is displaced in the MD direction.
In this example, one of the heads, the head 11, has the same function as the foregoing first reference example. In other words, the head 11 applies hot-melt adhesive that is for joining the continuous sheet member 2 and the rubber threads 5 and 5, the rubber threads 5 and 5 forming leg gathers. On the other hand, the other head, the head 11b, intermittently forms in the MD direction HMA applying areas 9b, 9b ... which is for joining the continuous sheet member 2 and rubber threads (not shown), the rubber threads forming waist gathers, for example.

=== Other Embodiments ===

While the reference examples and embodiments according to the invention are described above, the invention is not limited to the embodiments, etc and the invention can be altered as described below.
In the first reference example mentioned above, all the valves 14, 14, ... included in one head 11 share the same adjustment value Ya. However, this invention is not limited thereto. For example, the configuration may be altered so that several of the valves 14, 14, ... are selected from the control panel 40 and the adjustment value Ya is applied only to the selected valves 14, 14, .... In other words, the PLC 30 may be configured so that only the first regulation value, etc of the selected valves 14, 14, ... are shifted by the adjustment value Ya.
In the first embodiment mentioned above, all the valves 14, 14, ... included in one head 11 share the pair of the compensation-value tables for the opening and closing operations, and thereby all the valves 14, 14, ... share the pair of shared compensation values for the opening and closing operations. However, this invention is not limited thereto. For example, the configuration may be altered so that several of the valves 14, 14, ... are selected from the control panel 40, the pair of the compensation-value tables are applied to only the selected valves 14, 14, ..., and only the selected valves 14, 14, ... is compensated by using the pair of shared compensation values. In other words, the PLC 30 may be configured so that the first and second regulation values of the selected valves 14, 14, ... are each compensated based on the shared compensation value for opening and the shared compensation value for closing respectively.
In the foregoing first reference example, the first and second regulation values are provided as examples. However, as a matter of course, as shown in FIG. 1B, if there is the valve 14 which forms two applying areas 9f and 9f along the MD direction in the product pitch P, a third regulation value for the valve-opening signal of that valve 14 is set up and also a fourth regulation value for the valve-closing signal is set up, in addition to the first and second regulation values. It should be noted that in the case of three or more applying areas 9, 9, 9 ... in the MD direction, the number of the regulation values increases accordingly.
In the second reference example and the first embodiment mentioned above, the speedometer is included in order to measure the transport velocity V2 of the continuous sheet member 2. However, this invention is not limited thereto. For example, it is acceptable that the PLC 30 executes operations based on the following formula 6, thereby the transport velocity V2 is calculated based on the time interval $\Delta T$ at which the encoder 80 outputs the digital value. However, in this case, the operational load of the PLC 30 increases. Therefore, the speedometer is preferably included.

$$V2 = \Delta D / \Delta T \quad \ldots \quad (6)$$

The $\Delta D$ in the foregoing formula 6 means the amount by which the continuous sheet member 2 is transported from the time of output of a certain digital value (e.g., 8190) to the time of output of the next digital value (e.g., 8191), and the $\Delta D$ is a known value which each encoder has.

In the foregoing reference examples and embodiments, a diaper is described as an example of the absorbent article. However, this invention is not limited thereto as long as an article absorbs such exudates as urine, menstrual blood, etc. For example, a sanitary napkin may also be employed.

In the foregoing reference examples and embodiments, as an example of the encoder 80, an encoder which outputs a digital value at each rotation through a certain angle is provided. However, this invention is not limited thereto. For example, an encoder which generates a pulse at each rotation through a certain angle and outputs a reset signal at each rotation through the corresponding angle to the product pitch P (e.g. one turn) may be employed. In this case, the PLC 30 counts the number of the pulse output from the encoder, and resets the count to zero each time when receiving a reset signal. Thereby, the encoder cooperates with the PLC 30 and functions in the same manner as the foregoing encoder 80.

In the foregoing reference examples and embodiments, the hot-melt adhesive is provided as an example of "fluid". However, the invention is not limited thereto as long as the fluid is a fluid such as a liquid or a gel having a sufficient flowability to be intermittently discharged towards the continuous sheet member 2 associated with an absorbent article. Another type of adhesive may be employed, and any fluid other than adhesive may also be employed.

In the foregoing reference examples and embodiments, the non-contact discharge opening in which the tip of nozzle N, serving as a discharge opening, is not in contact with the continuous sheet member 2 is described. That is, the tip of the nozzle N is positioned with a spacing between the continuous sheet member 2. However, the invention is not limited thereto. A contact discharge opening may be employed. In other words, the tip of the nozzle N or a member disposed on the tip may be in contact with the continuous sheet member 2. As an example of the contact discharge opening, the following configuration can be provided: the tip of the nozzle N is provided with a sphere which can rotate like a ball of a ballpoint pen, the sphere is moved by keeping in contact with the continuous sheet member 2.

In the second embodiment mentioned above, the configuration including two heads 11 and 11b are provided as an example of a plurality of the heads 11. However, the number of the heads is not limited to two. Another head may be placed at a different position in the MD direction in the manufacturing line. As a matter of course, in this case, each head may have its own adjustment value Ya and its own pair of the compensation-value tables in the same manner as the second embodiment. Depending on the case, the adjustment value Ya and the pair of the compensation-value tables may be shared by all of the heads 11, 11b, .... With such a configuration, using the adjustment value Ya and the pair of the compensation-value tables, the discharging timing can be shifted for all of the valves 14, 14, ... included in all of the heads 11, 11b, ... so that the application pattern is displaced in the MD direction.

In the foregoing reference examples and embodiments, the nozzles N correspond to the valves 14 respectively. However, this invention is not limited thereto. For example, a plurality of nozzles N may correspond to one of the valves.

Reference Signs List

2 continuous sheet member for top sheet (continuous sheet member),
3 continuous sheet member for back sheet,
5 rubber thread, 7 continuous body of half-completed product,
9 applying area, 9b applying area, 9f applying area,
10 HMA applying device (fluid discharging device),
11 head, 11b head,
13 branch path, 14 valve, 15 solenoid valve,
30 PLC (controller), 40 control panel,
60 rubber-thread supply device, 61 arm,
70 pressing roller, 70a roll, 70b roll,
80 rotary encoder,
N nozzle (discharge opening), BL boundary position,

S1 HMA application section, S2 processing section

**Claims**

1. A fluid discharging device that discharges fluid from a plurality of discharge openings towards a continuous sheet member (2) which is continuously transported in a transporting direction, the discharge openings being arranged in a width direction of the continuous sheet member (2) associated with an absorbent article, comprising:

    a plurality of valves (14) that correspond to the discharge openings and intermittently discharge the fluid from the discharge openings by opening and closing operations; and
    a controller (30) that controls the opening and closing operations of each of the valves (14) individually depending on a transportation amount of the continuous sheet member, and
    a memory that stores a shared compensation value that is represented by a value indicating the transportation amount, wherein
    the controller (30) calculates the shared compensation value, based on a transport velocity of the continuous sheet member, and the controller (30) shifts an open-close timing of at least several valves of the plurality of valves (14) from a previously-determined open-close timing, based on the shared compensation value.

2. A fluid discharging device according to claim 1, wherein for each of the opening operation and the closing operation, the controller (30) has compensation data that represents a relationship between the transport velocity and the shared compensation value,
    in the opening operation, the controller obtains the shared compensation value for the opening operation of the several valves (14) based on the compensation data for the opening operation, and in the closing operation, the controller (30) obtains the shared compensation value for the closing operation of the several valves (30) based on the compensation data for the closing operation.

3. A fluid discharging device according to claim 1 or 2, wherein
    the previously-determined open-close timing includes a previously-determined opening timing and a previously-determined closing timing,
    the controller (30) includes, for each of the valves (14),
    a first regulation value that sets up the previously-determined opening timing and
    a second regulation value that sets up the previously-determined closing timing,
    the first regulation value and the second regulation value are represented by the value indicating the transportation amount, and
    using the value indicating the transportation amount, the controller (30) controls the opening and closing operations of each of the valves (14) individually depending on the transportation amount.

4. A fluid discharging device according to any one of claims 1 to 3, wherein all of the plurality of valves (14) are disposed in one head that includes the plurality of discharge openings, and
    the number of the several valves (14) is the same as the number of the plurality of valves.

5. A fluid discharging device according to any one of claims 1 to 4, wherein one valve (14) is provided for every discharge opening.

6. A fluid discharging method for dischargeing fluid from a plurality of discharge openings towards a continuous sheet member (2) which is continuously transported in a transporting direction, the discharge openings being arranged in a width direction of the continuous sheet member (2) associated with an absorbent article, comprising:

    preparing a plurality of valves (14) that correspond to the discharge openings and intermittently discharge the fluid from the discharge openings by opening and closing operations; and
    controlling the opening and closing operations of each of the valves (14) individually depending on a transportation amount of the continuous sheet member,
    calculating the shared compensation value that is represented by a value indicating the transport the transport amount, based on a transport velocity of the continuous sheet member, and
    storing the shared compensation value, and
    shifting an open-close timing of at least several valves of the plurality of valves (14) from a previously-determined open-close timing, based on the shared compensation value.

**Patentansprüche**

1. Eine Fluidaustragsvorrichtung, die Fluid von einer Vielzahl von Austragsöffnungen in Richtung eines kontinuierlichen Lagenelements (2), das kontinuierlich in einer Transportrichtung transportiert wird, austrägt, die Austragsöffnungen sind in einer Breitenrichtung von dem kontinuierlichen Lagenelement (2), das mit einem absorbierenden Artikel verknüpft ist, angeordnet, umfassend:

   eine Vielzahl von Ventilen (14), die zu den Austragsöffnungen korrespondieren und intermittierend das Fluid von den Austragsöffnungen durch Öffnungs- und Schließvorgänge austragen; und

   ein Steuergerät (30), das die Öffnungs- und Schließvorgänge jedes der Ventile (14) individuell steuert, abhängig von einer Fördermenge des kontinuierlichen Lagenelements, und

   einen Speicher, der einen geteilten Kompensationswert, der durch einen Wert, der die Fördermenge anzeigt, dargestellt wird, speichert, wobei

   das Steuergerät (30) den geteilten Kompensationswert berechnet, basierend auf einer Transportgeschwindigkeit des kontinuierlichen Lagenelements, und das Steuergerät (30) eine Öffnungs-Schließ-Terminierung von wenigstens mehreren Ventilen der Vielzahl von Ventilen (14) von einer vorher bestimmten Öffnungs-Schließ-Terminierung verändert, basierend auf dem geteilten Kompensationswert.

2. Eine Fluidaustragsvorrichtung gemäß Anspruch 1, wobei für jeden der Öffnungsvorgang und der Schließvorgang, das Steuergerät (30) Kompensationsdaten aufweist, die ein Verhältnis zwischen der Transportgeschwindigkeit und dem Kompensationswert abbilden,

   bei dem Öffnungsvorgang, das Steuergerät den geteilten Kompensationswert für den Öffnungsvorgang der mehreren Ventile (14), basierend auf den Kompensationsdaten für den Öffnungsvorgang, ermittelt, und bei dem Schließvorgang, das Steuergerät (30) den geteilten Kompensationswert für den Schließvorgang der mehreren Ventile (30), basierend auf den Kompensationsdaten für den Schließvorgang, ermittelt.

3. Eine Fluidaustragsvorrichtung gemäß Anspruch 1 oder 2, wobei
   die vorher bestimmte Öffnungs-Schließ-Terminierung eine vorher bestimmte Öffnungs-Terminierung und eine vorher bestimmte Schließ-Terminierung umfasst,
   das Steuergerät (30), für jedes der Ventile (14),
   einen ersten Regelwert, der die vorher bestimmte Öffnungs-Terminierung konfiguriert, und
   einen zweiten Regelwert, der die vorher bestimmte Schließ-Terminierung konfiguriert, umfasst,
   der erste Regelwert und der zweite Regelwert durch den Wert, der die Fördermenge anzeigt, dargestellt werden, und
   mittels des Werts, der die Fördermenge anzeigt, das Steuergerät (30) die Öffnungs- und Schließvorgänge jedes der Ventile (14) abhängig von der Fördermenge einzeln steuert.

4. Eine Fluidaustragsvorrichtung gemäß einem der Ansprüche 1 bis 3, wobei alle der Vielzahl von Ventilen (14) in einem Kopf, der die Vielzahl von Austragsöffnungen umfasst, angeordnet sind, und
   die Anzahl der mehreren Ventile (14) die Gleiche ist wie die Anzahl der Vielzahl von Ventilen.

5. Eine Fluidaustragsvorrichtung gemäß einem der Ansprüche 1 bis 4, wobei ein Ventil (14) für jede Austragsöffnung vorgesehen ist.

6. Ein Fluidaustragsverfahren zum Fluidaustragen von einer Vielzahl von Austragsöffnungen in Richtung eines kontinuierlichen Lagenelements (2), das kontinuierlich in einer Transportrichtung transportiert wird, die Austragsöffnungen sind in einer Breitenrichtung von dem kontinuierlichen Lagenelement (2), das mit einem absorbierenden Artikel verknüpft ist, angeordnet, umfassend:

   Vorbereiten einer Vielzahl von Ventilen (14), die zu den Austragsöffnungen korrespondieren und intermittierend das Fluid von den Austragsöffnungen durch Öffnungs- und Schließvorgänge austragen; und

   Steuern der Öffnungs- und Schließvorgänge jedes der Ventile (14) einzeln, abhängig von einer Fördermenge des kontinuierlichen Lagenelements,

   Berechnen des geteilten Kompensationswerts, der durch einen Wert, der den Transport die Fördermenge anzeigt, dargestellt wird, basierend auf einer Transportgeschwindigkeit des kontinuierlichen Lagenelements, und

   Speichern des geteilten Kompensationswerts, und

   Verändern einer Öffnungs-Schließ-Terminierung von wenigstens mehreren Ventilen der Vielzahl von Ventilen (14) von einer vorher bestimmten Öffnungs-Schließ-Terminierung, basierend auf dem geteilten Kompensationswert.

**Revendications**

1. Dispositif de distribution de fluide qui distribue un fluide à partir d'une pluralité d'ouvertures de distribution vers un élément de type bande continue (2) qui est transporté de manière continue dans une direction de transport, les ouvertures de distribution étant agencées dans une direction de largeur de l'élément de type bande continue (2) associé à un article absorbant, comprenant :

   une pluralité de valves (14) qui correspondent aux ouvertures de distribution et distribuent de manière intermittente le fluide à partir des ouvertures de distribution par des opérations d'ouverture et de fermeture ; et
   un dispositif de commande (30) qui commande les opérations d'ouverture et de fermeture de chacune des valves (14) individuellement en fonction d'une quantité de transport de l'élément de type bande continue, et une mémoire qui stocke une valeur de compensation partagée qui est représentée par une valeur indiquant la quantité de transport, dans lequel
   le dispositif de commande (30) calcule la valeur de compensation partagée, sur la base d'une vitesse de transport de l'élément de type bande continue, et le dispositif de commande (30) décale un rythme d'ouverture-fermeture d'au moins plusieurs valves de la pluralité de valves (14) à partir d'un rythme d'ouverture-fermeture déterminé précédemment, sur la base de la valeur de compensation partagée.

2. Dispositif de distribution de fluide selon la revendication 1, dans lequel pour chacune de l'opération d'ouverture et de l'opération de fermeture le dispositif de commande (30) a des données de compensation qui représentent une relation entre la vitesse de transport et la valeur de compensation partagée, lors de l'opération d'ouverture, le dispositif de commande obtient la valeur de compensation partagée pour l'opération d'ouverture des plusieurs valves (14) sur la base des données de compensation pour l'opération d'ouverture, et lors de l'opération de fermeture, le dispositif de commande (30) obtient la valeur de compensation partagée pour l'opération de fermeture des plusieurs valves (30) sur la base des données de compensation pour l'opération de fermeture.

3. Dispositif de distribution de fluide selon la revendication 1 ou 2, dans lequel le rythme d'ouverture-fermeture déterminé précédemment inclut un rythme d'ouverture
   déterminé précédemment et un rythme de fermeture déterminé précédemment, le dispositif de commande (30) inclut, pour chacune de valves (14),
   une première valeur de commande qui établit le rythme d'ouverture déterminé précédemment et
   une seconde valeur de commande qui établit le rythme de fermeture déterminé précédemment,
   la première valeur de commande et la seconde valeur de commande sont représentées par la valeur indiquant la quantité de transport, et
   en utilisant la valeur indiquant la quantité de transport, le dispositif de commande (30) commande les opérations d'ouverture et de fermeture de chacune des valves (14) individuellement en fonction de la quantité de transport.

4. Dispositif de distribution de fluide selon l'une quelconque des revendications 1 à 3, dans lequel la totalité de la pluralité de valves (14) sont disposées dans une tête qui inclut la pluralité d'ouvertures de distribution, et le nombre des plusieurs valves (14) est le même que le nombre de la pluralité de valves.

5. Dispositif de distribution de fluide selon l'une quelconque des revendications 1 à 4, dans lequel une valve (14) est fournie pour chaque ouverture de distribution.

6. Procédé de distribution de fluide pour distribuer un fluide à partir d'une pluralité d'ouvertures de distribution vers un élément de type bande continue (2) qui est transporté de manière continue dans une direction de transport, les ouvertures de distribution étant agencées dans une direction de largeur de l'élément de type bande continue (2) associé à un article absorbant, comprenant :

   la préparation d'une pluralité de valves (14) qui correspondent aux ouvertures de distribution et distribuent de manière intermittente le fluide à partir des ouvertures de distribution par des opérations d'ouverture et de fermeture ; et
   la commande des opérations d'ouverture et de fermeture de chacune des valves (14) individuellement en fonction d'une quantité de transport de l'élément de type bande continue,
   le calcul de la valeur de compensation partagée qui est représentée par une valeur indiquant la quantité de transport, sur la base d'une vitesse de transport de l'élément de type bande continue, et

le stockage de la valeur de compensation partagée, et

le décalage d'un rythme d'ouverture-fermeture d'au moins plusieurs valves de la pluralité de valves (14) à partir d'un rythme d'ouverture-fermeture déterminé précédemment, sur la base de la valeur de compensation partagée.

FIG. 1A

FIG. 1B

FIG. 2

FLOWING DIRECTION OF
OPERATIONAL FLUID
OF SOLENOID VALVE

VALVE CLOSE

VALVE OPEN

FIG. 3

EP 2 522 322 B1

MD DIRECTION

DOWNSTREAM ◄————————————— UPSTREAM

2

9

BL

BL

CD
DIRECTION

L1

L2

P

P

FIG. 4

MD DIRECTION

FIG. 5A

9

9

$\delta$ a

BL

BL

CD
DIRECTION

11

$\delta$ a

$\delta$ a

FIG. 5B

BL

BL

CD
DIRECTION

11

FIG. 6

FIG. 7

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6237957 A
- EP 1591170 A2
- US 20060108513 A1